# EUROPEAN PATENT APPLICATION

(11) **EP 3 831 814 A1**
(43) Date of publication of application: **09.06.2021**
(21) Application number: 20178119.2
(22) Date of filing: 03.06.2020
(51) Int. Cl.: C07D 265/16, C09J 161/34

(54) **A BENZOXAZINE ADHESIVE FOR POLYETHYLENE AND THE PREPARATION AND APPLICATION METHOD THEREOF**

(30) Priority: 03.12.2019 CN 201911223519
(71) Applicant: Shandong University, Jinan, Shandong 250199 (CN)
(72) Inventor: LU, Haifeng, Jinan, Shandong 250199 (CN); SUN, Anbang, Jinan, Shandong 250199 (CN); LIU, Shaojie, Jinan, Shandong 250199 (CN); FENG, Shengyu, Jinan, Shandong 250199 (CN); SHAN, Yajuan, Jinan, Shandong 250199 (CN); WEI, Zengyue, Jinan, Shandong 250199 (CN); LI, Cuiyun, Jinan, Shandong 250199 (CN); ZENG, Qingming, Jinan, Shandong 250199 (CN); DONG, Zhichao, Jinan, Shandong 250199 (CN)
(74) Representative: Proi World Intellectual Property GmbH

(57) **Abstract**

The invention discloses a benzoxazine adhesive for polyethylene and the preparation and application method thereof. The adhesive is a phenolic hydroxyl group and long-chain alkyl group-containing benzoxazine compound prepared with the method of preparing benzoxazine group-containing long-chain alkanes from the amino group and long chain alkyl group-containing organic compounds through one step reaction. The preparation method of the invention has simple and controllable synthesis conditions and high synthesis efficiency. The benzoxazine group-containing long-chain alkane synthesized with the method described in the invention can be used as an adhesive with high adhesion strength directly; or used as a basic ingredient to produce another adhesive after being added in other fillers so as to meet the different needs of various special occasions. The invention has combined the hydrogen-bond interaction, the compatibility between the long-chain alkyl group and the polyethylene, and the benzoxazine component skillfully. It has realized the preparation of an adhesive with high adhesion strength relying on the no volume shrinkage, high adhesive strength and other characteristics of the benzoxazine during the polymerization and combined with the adhesive properties provided by the hydrogen-bond interaction.

## Description

### Technical Field

The invention relates to an adhesive for polyethylene and the preparation and application method thereof, and belongs to the field of high polymer material preparation and application.

### Background Art

Polyethylene is a kind of high-performance general-purpose plastics obtained through ethylene homopolymerization. It has a variety of features, including nontoxicity, readily available raw materials, cheaper cost, high mechanical strength, and good heat resistance and chemical stability. It also has its advantages even when compared to metal materials, such as excellent insulating properties, corrosion resistance and molding processability. The polyethylene materials are widely used in agriculture, packaging industry, automobile industry, construction industry, printing, electronics and other fields in forms of film, sheet, various injection molded products and the like. The polyethylene products, in many cases, also need to go through bonding, electroplating, coating, printing and other secondary processing means before being used, so a special adhesive for polyethylene become increasingly required.

### (1) Adhesive for polyethylene film

The film-mulching cultivation technology uses the polyethylene film as shed film and mulching film in the agriculture, and has created great economic and social benefits. Research and development of the long-life and multi-function polyethylene double-layer inflatable greenhouse and its supporting technologies can do a favor to the vegetable cultivation in winter in northern China, thus improving the production and application level of agricultural shed film in China. The application of mulching films for dams and landfills has guaranteed the project quality and avoided the leak. However, the problems caused thereby are also very significant and need to be solved urgently. Along with the widespread applications of the polyethylene film in agriculture, package and other industries, the damage and repair problem of the film during the use has become a focused issue. Therefore, a special adhesive is in urgent need to bond the polyethylene films

### (2) Adhesive for metal/plastic/metal composite laminate

Metal/plastic/metal composite laminate materials are of great significance to the lightweight, heat insulation, damping vibration attenuation and other indicators of the traffic vehicles, aircrafts, electronic equipment and other equipment and facilities. The adhesive strength between the metal plate and the PE coiled material relates to the application prospects of the composite material. Similar problems also exist in the oil and gas pipelines. The normal operation of the oil and gas pipelines have been significantly affected due to the ubiquitous poor adhesion properties of the anticorrosive coating and joint coating technologies that take polyethylene as a major component. After the pipelines being buried underground, under the action of the pipeline stress and the shear force caused by soil flow, certain cracks will occur between the thermal shrinkage polyethylene sheet and the polyethylene coating due to the poor adhesive effects between the thermal shrinkage polyethylene bushing and the polyethylene coating. Then, water and other corrosive substances may enter the weld through the cracks, causing corrosion to the metals and further oil and gas leak accidents and great economic losses. Therefore, the adhesive properties between the polyethylene and metals are even more important.

### (3) Adhesive for ultra-high molecular weight polyethylene products

The ultra-high molecular weight polyethylene is a kind of thermoplastic engineering plastic with a linear structure and excellent comprehensive performance, including excellent impact resistance, excellent chemical resistance, excellent stress cracking resistance and the like. It has the potential of replacing a variety of metal materials, and has been widely applied in textile, chemical industry, machinery, aerospace, military industry and other fields. Particularly, in the current materials market, for purposes of improving the corrosion resistance of the materials and reducing the load bearing, it has become an irresistible trend to replace steel with plastics. Used in high-speed trains and mainly in rail fasteners, damping material, internal decoration, electrical parts and the like, the ultra-high molecular weight polyethylene can effectively reduce the noise and vibration during locomotive running, reduce the body weight and improve the riding comfort. During installation, it is conductive to the formation of good waterproof seal joints by fixing the materials with the adhesion method in replacement of the traditional welding process, and can improve the jointing and sealing quality of the train. During use, adhesion between two polyethylene parts, or one polyethylene part and another plastic or metal part is also inevitable.

### (4) Adhesive for ultra-high molecular weight polyethylene fibers

Ultra-high molecular weight polyethylene fibers, the world's third generation of high-performance fibers, have super high strength, high modulus, low density, corrosion resistance, impact resistance and other excellent properties, and have extremely important applications in national defense and military industry, aerospace and other high-tech industries. For example, the ultra-high molecular weight fiber reinforced composite material has been used in radome, helicopter bulletproof armor and other products. However due to their poor bonding properties with adhesives, applications of the ultra-high molecular weight polyethylene fibers in the field of composite materials have been affected to a great extent.

There are a lot of researches that have analyzed the non-stick properties of the polyethylene. According to the researches, such non-stick properties are caused by a variety of reasons, mainly including:
(1) Polyethylene molecules are of linear long-chain structure that comprises a large number of nonpolar methylene groups. The molecular chains contain no polar functional groups or reactive functional groups, and are chemically inert and hard to form chemical bonds with various adhesives. Thus, the adhesive force of the adhesive interface of the polyethylene material mainly comes from the intermolecular secondary bond forces, wherein the orientation force, induction force and hydrogen bond are all related to the polarity of the molecule. However, as polyethylene molecules are nonpolar molecules, only a small secondary bond force can be formed at the contact interface of the polyethylene and the adhesive. Therefore, the adhesive can only have a relatively small adhesive force on the surface of the polyethylene relying on the dispersion effects, forming a low bonding strength.
(2) The polyethylene with high crystallinity can hardly form intermolecular winding with the adhesive through surface swelling. Due to a high relative molecular mass, the interchain Van der Waals' force of the polyethylene molecules holds the adjacent molecular chains close to each other and make them arranged neatly, resulting in high crystallinity. High-crystallinity high polymers are generally hard to have swelling or dissolution, or form molecular chain diffusion, so they can hardly provide good adhesive joints or good adhesive properties.
(3) The polyethylene has a weak boundary layer. The polyethylene is generally to be mixed with stabilizer, plasticizer, release agent and other low relative molecular mass compounds while being processed. As time goes by, the plasticizer and other low molecular weight compounds will volatilize and exude to the surface, resulting in a continuous decrease in adhesion strength. Meanwhile, the plasticizer in the adherend will also seep into the adhesive layer, making the adhesive soften and lose the cohesion adhesion strength. The plasticizer will also concentrate on the interface to form a weak boundary layer, which can reduce the adhesion strength and even lead to the adhesion interface separation in severe cases.
(4) Low surface energy of the polyethylene makes the adhesive unable to wet its surface. A solid surface can be fully wetted to obtain a higher adhesive strength only when the surface tension of the adhesive is less than the critical surface tension of the bonded solid surface. However, the commercially available adhesives all have higher surface tension than that of the polyethylene, such that the adhesives cannot wet the polyethylene fully, resulting in the poor adhesion between the two. Therefore, bubbles are easy to remain in the joint boundary layer when the polyethylene is hard to be wetted by the adhesive liquid. Then, the joint is prone to stress concentration when under stress, thus resulting in joint damage even under a small load.

Despite that the polyethylene is difficult to get bonded due to the above four characteristics, considering the higher demands of the industrial applications on the adhesive properties of the polyethylene, there are a lot of researches that keep focusing on the adhesive performance of the polyethylene and have proposed a variety of improvement methods. Among them, the fusion-based hot welding technology was first developed.

For polyethylene, the fusion welding method is first recommended. The easiest way to connect two pieces of polyethylene materials is to heat and melt the joint part. However, as fully cross-linked polyethylene is infusible, such method cannot be used for cross-linked polyethylene. This method can achieve good connection effects only when used for uncross-linked or lightly cross-linked polyethylene. The FUSION-WELD system produced by the TAPECOAT Company and the ELECTROFUSION system produced by the ISCO Industries both adopt the direct hot welding technology. The FUSABOND system produced by the DuPont Company uses maleic anhydride-grafted polyethylene as an intermediate layer for hot welding. There are also some technologies that use hot-melt adhesives, but they are used at very high temperatures. To sum up, all these technologies are expensive generally. Furthermore, as the polyethylene is a bad conductor of heat, a strong residual stress may exist in the polyethylene after hot welding. Therefore, the fusion welding method is not widely used.

In addition to the hot welding technology, there are also a number of studies that attempt to improve the adhesive performance of the polyethylene from the perspective of adhesive interface. From the perspective of adhesive interface, they can be divided into three types:
(1) The polyethylene itself is modified.
   This method is mainly to introduce polar groups into the main chain of the polyethylene through copolymerization so as to improve the surface polarity of the polyethylene material. However, as modification of the polyethylene itself has essentially deviated from the inherent advantages and characteristics of the material and the original advantages of the material, such as low cost, will become all gone, this method is rarely used.
(2) Surface of the polyethylene is to be treated to improve its surface polarity and energy.

The surface treatment methods mainly include physical machining and chemical modification. Among them, the physical machining includes roughness machining and solvent method, while chemical modification can be further divided into surface oxidation and surface grafting. All these methods are to be introduced in turn as follows:
① Roughness machining
   Roughness machining methods include sand blasting, quartz sand grinding, "flame spraying and baking + mechanical roughening" and the like. Such methods can do little benefit to improving the adhesive effects of the polyethylene and the epoxy resin, and may damage the integrity of the PE layer.
② Solvent treatment
   The solvent treatment method is to pre-treat the polyethylene with the solvents, such as benzene, methylbenzene, decalin and trichloroethylene or perchloroethylene. The research that uses dimethylbenzene for surface treatment of the ultra-high molecular weight polyethylene fiber has shown that, the contact area between the fiber and the resin can be increased by dissolving the surface amorphous region and part of the crystalline region of the fiber to produce surface etching, unevenness and surface roughness, thus improving the adhesive properties of the fiber. As time goes on, the crystallinity and melting point of the fiber decrease gradually, indicating that the solvent molecules have infiltrated into the fiber, weakened the interactions between macromolecules and dissolved the amorphous region and part of the crystal region. Due to the reduced crystallinity, the strength of the fiber decreases. However, for environmental reasons, the method has also been gradually abandoned.
③ Surface coating
   The surface coating method is a process of applying an affinity layer (polyvinyl alcohol, polyvinyl acetate alcohol or celluloid ether) to the surface of the polyethylene during extrusion molding. With the increase of the content of polyvinyl acetate alcohol, the adhesive properties of the polyethylene can be improved significantly, while the breaking strength of the fiber will be reduced obviously. Therefore, it is not feasible to improve the adhesive properties of the material simply by increasing the blending ratio. There are also a number of studies that attempt to have the organic or inorganic (glass and asbestos) fabrics fused into the surface of the polyethylene. Then, the fabric layer of the polyethylene can be bonded with epoxy resin adhesive. However, there are still many difficulties in lining a container with such a thin layer.
④ Surface oxidation
   The surface oxidation method is to have the surface of the polyethylene oxidized first, and then have the oxidized surface to react with the adhesive. Relatively mature surface oxidation pretreatment technologies include:
   A. Flame oxidation: The method is to pre-treat the material with flame heating, and has high requirements on the operation techniques.
   B. Corona discharge: The method is to pretreat the material through the discharge process. However, studies have shown that the corona discharge alone can do limited help to improving the adhesion strength.
   C. Plasma method: The method is to treat the surface of the polyethylene with plasma. This method does well in improving the adhesive properties of the polyethylene, but the equipment required by it has poor continuation degree and stability. Additionally, the active groups on the surface of the plasma have a decay rate after treating the ultra-high molecular weight polyethylene, reaching up to 1/3 in 2h. Also, this method has high requirements on production line operation techniques.
   D. Acid treatment method: the method is to use concentrated acid water solutions (such as HCl, HBr, HI, HF and H2SO4) for pre-treatment.
   E. Halogenation method: This method to realize halogenation for the polyethylene with halides, halogens and other substances. For example, the direct fluorination method is a new method of surface modification for high polymers that uses fluorine gas as the fluorination reagent directly. After fluorination, there will be nano-sized fluorinated layers formed on the surfaces of the high polymers, which can have the adhesiveness and other properties of the material improved greatly whiling maintaining the mechanical properties of the material itself. This method has a lot of advantages, including low cost, no influence on the properties of the material itself, being able to produce chemical reactions with the surfaces of most polymers, flexible process selection, no need of catalyst, being able handle all kinds of geometric products and the like, and is the surface modification method developed rapidly in recent years. However, due to the high toxicity of the fluorine gas, this method has higher requirements on the air tightness of the equipment and the worker's operation techniques.
   F. Chemical oxidation method: This method uses chromic acid lotion, nitric acid, mixed acid of nitric acid and sulfuric acid, sodium hypochlorite + acetic acid, hydrogen peroxide + ozone and other chemical reagents for oxidation. Studies have shown that the chromic acid lotion treatment for the surface of the polyethylene fiber aims mainly to dissolve the amorphous region on the surface of the fiber, so that the surface of the fiber will produce rough marks and flake-like bumps, and the adhesion between the polyethylene fiber and the epoxy resin can be increased greatly relying on such complex geometric shapes. During the chromic acid treatment, some surfaces of the fibers also undergo oxidation reactions, introducing polar groups into the surfaces of the fibers following an oxidation mechanism as follows: an intermediate product of tetravalent chromium is produced first and then is hydrolyzed into alcohols; and the alcohols are further transformed into alkenes, aldehydes, ketones or carboxyl groups through oxidation and chain scission. As generally recognized, the introduction of polar groups can have the wettability of the fiber improved; and oxidation of certain surfaces and production of macromolecular split products can have the mutual attraction and mutual diffusion between the adhesive interfaces strengthened, which will improve the adhesive properties then. As can be seen, the surface treatment of the chromic acid lotion can realize both physical modification (surface etching) and chemical modification (introducing oxygen-containing groups into the chain of macromolecules) for the surface of the fiber. Therefore, the chromic acid lotion treatment can play a better role in improving the adhesive properties of the polyethylene fiber. However, the chromium-containing wastewater produced thereby can do greater harm to both the environment and the workers.
⑤ Surface grafting
   A. Chemically induced grafting: this method is to graft some polymers with reactive groups to the surface of the polymer, and then uses certain adhesives that can react with such reactive groups to realize adhesion. Because they are joined together by chemical bonds, a very high bonding strength (shear strength up to 17 kg/cm²) can be obtained theoretically. However, due to complex processes and long reaction time, this method still has not been applied in practice.
   B. Radiation grafting: This method is to graft some polar groups to the surface of the polymer relying on the high energy radiation technology so as to increase the surface energy of the polymer, and then uses the adhesives to realize adhesion. This method can greatly improve the surface adhesiveness of the polymer, but it cannot realize a high adhesive strength relying on the physical action alone.
   C. UV grafting: This method is to realize the grafting relying on the interaction of the ultraviolet light with the polymer surface. The ultraviolet grafting can be restricted to the polymer surface strictly without damaging the polymer itself, and has also a number of advantages, including being simple and easy to use, low equipment cost and being easy to realize continuous production. Therefore, it has become the fastest developed surface modification technology recently (tensile shear strength up to 2.2MPa).

The above methods all have improved the adhesive properties of the polyethylene to some extent, but they are all complicated in operations and cannot realize stable enough effects, for reasons that they all need to treat the low energy surface with various methods, such as chemical, radiation, discharge and flame method, to increase the surface energy first, and then use ordinary adhesives to realize adhesion. Therefore, they are not quite applicable. The traditional chemical treatment methods do great harm to the environment. The plasma, ultraviolet oxidation, radiation, glow discharge and other surface treatment technologies all require specialized energy sources, and many require vacuum working conditions, so they are not economical, and relatively difficult to be widely used. Additionally, most of these technologies have their dead zones as follows: the shaded areas or the inner surface areas (such as the inner wall of the hole) of the complex-shaped parts (such as fiber bundle) are sheltered and cannot be treated, resulting in uneven surface treatment. Furthermore, due to the need for vacuum conditions, these technologies are also difficult to be applied to large-size or complex-shaped components.

### (3) Adhesives that do not need surface treatment

Due to the nonpolar properties and insolubility of the polyethylene, there is no known adhesive that can be used to achieve a satisfactory adhesive strength for the polyethylene. Currently, the mainstream researches both at home and abroad are all attempting to synthesize adhesives with similar structure and surface energy to polyolefin to realize direct adhesion. Hot melt adhesives and acrylic series adhesives are the two examples of them. Types and properties of the typical adhesives are shown in Table 1.

**Table 1 Typical adhesives for polyethylene**

| S/N | Type | Key indicator | Supplement |
|---|---|---|---|
| 1 | Acrylic adhesive | Peeling strength: 10N/cm | This type of adhesives take acrylic emulsion as the main component, and are mostly compound emulsion adhesives |
| 2 | .Polyurethane adhesive | Peeling strength: 2.08N/cm | Used for bonding of PE/PET composite films |
| 3 | Styrene- butadiene-styrene block copolymer (SBS) | Peeling strength: 0.67MPa | Dry the materials for a while after applying the adhesive, and join the adhesive interfaces together; apply a certain pressure by hands and cure the materials at room temperature for 24 hours before performance testing. |
| 4 | Pressure-sensitive adhesive | Peeling strength: 6.0N/cm | Apply the adhesive to the polyethylene tape with the hot melt pressure sensitive adhesive coater |
| 5 | Ethylene vinyl acetate (EVA) adhesive | Peeling strength: 8.0N/cm | Apply the adhesive evenly to the PE film, and cut the film into 2.5cm wide and 1.5cm long sample strips; and leave it at room temperature for 2 days |
| 6 | One package RTV silicon rubber | Tensile strength after curing: 3.1MPa; Elongation at break: 300% | One package RTV silicon rubber has good tensile strength, elongation at break and adhesiveness, and has well met the sealing and adhesion requirements of the PE shells and glass covers of novel automotive luminaires. |
| 7 | Chlorinated polypropylene adhesive | Peeling strength: 0.66MPa | Apply a layer of the adhesive evenly on the surface of the treated test pieces, and dry them for 3min around at room temperature; after the solvent evaporates and the adhesive does not stick to hand any more, join the 2 test pieces together tightly; put them into the oven for heading and curing after fixing and clamping them tightly; take them out after a certain period of time and cool them down to room temperature before testing their shear strength on a tensile test machine. |
| 8 | Graft neoprene adhesive | 3.12 KN/m | Weigh a certain amount of the adhesive solution, and apply it evenly on the PE plates evenly after mixing with 8%- 10% 7900 cross-linking agent; dry them for a certain time of period after two coatings and then join them together for curing |
| 9 | Butyl pressure sensitive adhesive for film | Peeling strength: 10.33N/cm; shear strength: 8.62N/cm² | The optimum proportions are w(butyl rubber) =5%, w (polyisobutylene)=10%, w (tackifying resin)=15% and w(carbon black)=2% |
| 10 | DP8005 produced by 3M Company | | It uses a trialkylborine as a free radical initiator to initiate the polymerization of acrylate monomers. Its curing time is too short, and it can be used for small parts bonding, but not for pipeline construction. |
| 11 | SG-P-10 Primer coating and modified α-cyanoacrylate quick - drying adhesive | Tensile shear strength: 8.0MPa and above; almost equaling to the breaking strength of the polyethylene material | However, its price is very high and currently is used only for polyethylene adhesion in special occasions. |

The above technologies have solved the surface adhesion problem of the polyethylene material to a certain extent, but the adhesion performance still needs to be further improved.

### Description of the Invention

In view of the poor surface adhesion performance of the polyethylene materials of the existing technologies, the invention provides a phenolic hydroxyl group-containing benzoxazine adhesive special for the surface adhesion of the polyethylene materials. The invention, starting from the amino-containing organic compounds, can have the phenolic hydroxyl group-containing benzoxazine adhesive prepared through only one step reaction. The preparation method of the invention has simple and controllable synthesis conditions and high synthesis efficiency. The phenolic hydroxyl group-containing benzoxazine adhesive synthesized with the method described in the invention can be used as an adhesive with high adhesion strength for the polyethylene material directly; or used as a basic ingredient to produce another adhesive after being added in other fillers; or used as a primer coating to realize the surface modification of the polyethylene material so as to meet the different needs of various special occasions.

### Summary of the Invention

The invention provides a benzoxazine adhesive for polyethylene and the preparation and application method thereof. The invention, starting from the amino-containing organic compounds, can have the phenolic hydroxyl group-containing benzoxazine adhesive prepared through only one step reaction. The phenolic hydroxyl group-containing benzoxazine adhesive prepared with the method described in the invention can be used as an adhesive directly; or used as a primer coating; or used as a basic ingredient to produce another adhesive after being added in other fillers to meet the different needs of various special occasions.

### Detailed Description of the Invention

The technical solution of the invention is as follows:
An adhesive for polyethylene, which is a phenolic hydroxyl group and long-chain alkyl group-containing benzoxazine compound

According to a preferred embodiment of the invention, the said adhesive is a compound with the structure as shown in chemical formula (I):

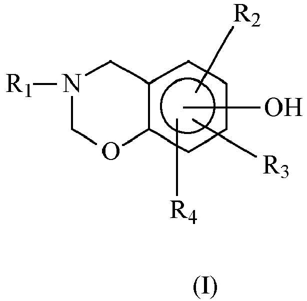

In chemical formula (I), R₁ is an organic group containing long-chain alkyl groups; R₂ to R₄ are various aliphatic hydrocarbon groups, silicon alkyl groups and/or aromatic hydrocarbon groups; or R₂ to R₄ are directly hydrogen atoms respectively.

According to an embodiment of the invention, the preparation method of the said adhesive for polyethylene comprises the steps as follows:
Under the conditions of with or without solvents, the amino group and long chain alkyl group-containing organic compound is mixed with the aldehyde (ketone) compound and the organic compound with no less than two phenolic hydroxyl groups in a benzene ring to react with each other to get a phenolic hydroxyl group- containing benzoxazine adhesive.

The corresponding reaction equation of the phenolic hydroxyl group-containing benzoxazine adhesive obtained in the invention is as follows:

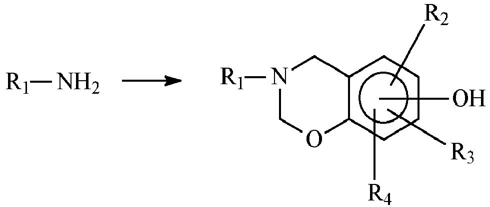

According to a preferred embodiment of the invention, the said amino group and long chain alkyl group-containing organic compound is an organic compound containing no less than one amino group and long-chain alkyl group simultaneously, including amino-containing small molecule compound, amino-containing linear polymer, amino-containing comb-like macromolecule, amino-containing dendrimer and amino-containing composite material; according to a further preferred embodiment of the invention, the amino-containing compound is small molecule compound containing multiple amino groups, linear polymer with amino groups in the side chain and amino-containing dendrimer;

According to a preferred embodiment of the invention, the said aldehyde (ketone) compounds are all kinds of aldehyde (ketone) organic compounds that can be dissolved in this system; according to a further preferred embodiment of the invention, the said aldehyde (ketone) compound is small molecule aldehyde (ketone) compound; according to a more preferred embodiment of the invention, the said aldehyde (ketone) compound is formaldehyde, acetone, benzaldehyde or cyclohexanone.

According to a preferred embodiment of the invention, the said organic compound with no less than two phenolic hydroxyl groups in a benzene ring is an organic compound whose molecular structure contains no less than one benzene ring and no less than two phenolic hydroxyl groups in a certain benzene ring; according to a further preferred embodiment of the invention, the said organic compound with no less than two phenolic hydroxyl groups in a benzene ring indicates all kinds of dihydric phenol compounds, trihydric phenol compounds and tetrahydric phenol compounds; according to a more preferred embodiment of the invention, the said organic compound with no less than two phenolic hydroxyl groups in a benzene ring is catechol, resorcinol, hydroquinone, phloroglucinol and four phenolic group benzene.

According to a preferred embodiment of the invention, the said solvents are all kinds of polar or non-polar solvents that can dissolve the reactants, but do not react with the reactants; according to a further preferred embodiment, they are methylbenzene, tetrahydrofuran, chloroform, methyl alcohol, diphenyl ether, dimethyl sulfoxide and N, N-dimethyl formamide; according to a more preferred embodiment, they are methylbenzene and tetrahydrofuran.

According to a preferred embodiment of the invention, the molar ratio of the said amino-containing organic compound, aldehyde (ketone) compound and organic compound with no less than two phenolic hydroxyl groups in a benzene ring is 1: (0.1-15): (0.1-20); according to a further preferred embodiment, the ratio is 1:(2-15):(1-10).

According to a preferred embodiment of the invention, the reaction temperature of the said amino-containing organic compound, aldehyde (ketone) compound and organic compound with no less than two phenolic hydroxyl groups in a benzene ring is 30-180°C, and is further preferred to be 40-90°C.

According to a preferred embodiment of the invention, the reaction time of the said amino-containing organic compound, aldehyde (ketone) compound and organic compound with no less than two phenolic hydroxyl groups in a benzene ring is 1-60h, and is further preferred to be 1-30h.

According to an embodiment of the invention, a benzoxazine adhesive composition for polyethylene comprises the following components in parts by mass:
100 parts of phenolic hydroxyl group and long-chain alkyl group-containing benzoxazine adhesive, 0- 10 parts of catalyst A, 0-400 parts of filler and 0- 200 parts of auxiliaries.

According to a preferred embodiment of the invention, the said catalyst A is a compound that can catalyze the ring opening and polymerization reaction of the benzoxazine, including the various generalized lewis acids and bases; according to a further preferred embodiment, it is benzenesulfonic acid, acetic acid and sodium hydroxide, and the dosage is preferred to be 0- 3 parts.

According to a preferred embodiment of the invention, the said filler is a variety of additives that can improve the performance of the benzoxazinyl adhesive, and is further preferred to be fumed silica, precipitated silica, carbon black, calcium carbonate, aluminium hydroxide and/or aluminium hydroxide as well as any of the above compounds that have been specially treated, and is more preferred to be silazane treated white carbon black. The dosage of the filler is 0- 400 pars and is preferred to be 0-30 parts.

According to a preferred embodiment of the invention, the said auxiliaries are the various auxiliaries that do not significantly reduce the performance of the adhesive after being added, including all kinds of functional components and non-functional components; according to a further preferred embodiment, they are thermal oxygen stabilizer, flame retardant, conductive agent, vesicant, deep curing agent, pigment and/or plasticizer; iron red is further preferred and the dosage is preferred to be 0-10 parts.

According to the invention, the application method of the said benzoxazine adhesive for polyethylene comprises the following steps:
The adhesive can be used as an adhesive directly;
Or used together with other catalysts, fillers and auxiliaries.

According to a preferred embodiment of the invention, when used together with other catalysts, fillers and auxiliaries, the phenolic hydroxyl group-containing benzoxazine adhesive is to be mixed with catalyst A, fillers and auxiliaries evenly as a basic ingredient; then, the mixture is to be applied to the adhesive interface to realize good adhesion through programed heating.

According to a preferred embodiment of the invention, the said temperature programming is a process of temperature rising from the initial temperature to the final temperature, with a temperature difference as the gradient and through multiple "temperature rising -constant temperature" links, wherein the said temperature difference is a conditionally controllable temperature gradient allowed by the adhesion conditions, including any temperature difference ranging from 5°C and 100°C, and is preferred to be 20°C; in the said "temperature rising -constant temperature" link, the temperature rising rate is an arbitrary heating rate between 0.1°C/min and 20°C/min, and is preferred to be 10°C/min; in the said "temperature rising -constant temperature" link, the constant temperature time ranges from 1 minute to 180 minutes and is preferred to range from 30 minutes to 120 minutes and more preferred to be 60 minutes; the said initial temperature is an arbitrary temperature value between the room temperature and the final temperature and is preferred to be 60°C; the said final temperature is a temperature value allowed by the adhesion conditions that can achieve the best adhesion performance of the adhesive under such adhesion conditions, including a certain optimum adhesion temperature between 60°C and 200°C obtained through practical experiments.

Steps not specified in details in the invention shall be according to the existing technologies.

The principle and beneficial effects of the invention are as follows:
The said adhesive for polyethylene in the invention is a phenolic hydroxyl group-containing benzoxazine compound. This compound can release a new phenolic hydroxyl group when turning into polybenzooxazine after being heated, so that the structure containing no less than two phenolic hydroxyl groups in a benzene ring can be formed. With the structure of containing two or more phenolic hydroxyl groups in a benzene ring, it will have strong adhesive properties, and thus can realize the adhesion of the polyethylene material. Particularly, as the structure with two or more phenolic hydroxyl groups in a benzene ring is formed in the bonding process, the compound does not have strong adhesive properties as a starting material, which thus facilitates the adhesion operations. Compared with the present invention, those compounds containing two or more phenolic hydroxyl groups in a benzene ring are inconvenient to be used as adhesives due to their high initial viscosity. Such difference indicates that the invention has achieved a significant advantage in convenience.

The reaction route of the invention has combined the hydrogen-bond interaction, the compatibility between the long-chain alkyl group and the polyethylene, and the benzoxazine component skillfully. It has realized the preparation of an adhesive with high adhesion strength relying on the no volume shrinkage, high adhesive strength and other characteristics of the benzoxazine during the polymerization and combined with the adhesive properties provided by the hydrogen-bond interaction. The compatibility between the long-chain alkyl group and the polyethylene is especially suitable for the surface treatment of the polyethylene and other non-stick materials. The reaction route of the invention has abandoned the previously reported scheme of using modified polyethylene as a surface adhesive for the polyethylene material, and has significantly improved the surface adhesion strength of the polyethylene material. Relying on simple and controllable synthesis conditions, high synthesis efficiency, simple adhesion operations and good adhesion properties, the invention has achieved remarkable effects.

The process of the invention comprises one step reaction. Compared to the two-step or multi-step reactions described in the related similar documents or patents, it has the characteristics, including simple steps, convenient operations, time saving and high efficiency. Particularly, such reaction can occur without the use of any solvent, but the use of solvent is helpful to improve the blending of materials and the reaction efficiency. Taking the polarity, boiling point, stability, safety and other aspects of the solvents into consideration, toluene and tetrahydrofuran are preferred. The invention is to introduce the phenolic hydroxyl groups into the structure of the benzoxazine relying on the formation process of the benzoxazine groups. Therefore, to improve the product conversion rate, it is necessary to appropriately increase the feeding ratio of the aldehyde (ketone) compound in this step, depending on the specific type of the aldehyde compound. When formaldehyde solution is used, a 10 times feeding ratio can be selected; when tripolyformaldehyde is used, a 2 times feeding ratio can be selected.

The benzoxazine adhesive for polyethylene synthesized in the invention can be used directly as an adhesive or as a primer coating, and can also be used to produce other adhesives after compounding with other substances. As the benzoxazine group has the advantage of zero volume contraction during ring-opening copolymerization, such adhesive realizes crosslinking mainly through the ring-opening copolymerization of the benzoxazine groups. Of course, the reactions of other reactive bonds in the system may also have partially participated in the crosslinking. The ring opening process of the benzoxazine groups is generally achieved by heating. In order to ensure that the adhesive has good adhesive properties, the method of temperature programming is generally used to realize the crosslinking. The temperature programming is a process of temperature rising from the initial temperature to the final temperature, with a temperature difference as the gradient and through multiple "temperature rising -constant temperature" links, wherein the said temperature difference is a conditionally controllable temperature gradient allowed by the adhesion conditions, including any temperature difference ranging from 5°C and 100°C, and is preferred to be 20°C; in the said "temperature rising -constant temperature" link, the temperature rising rate is an arbitrary heating rate between 0.1°C/min and 20°C/min, and is preferred to be 10°C/min; in the said "temperature rising -constant temperature" link, the constant temperature time ranges from 1 minute to 180 minutes and is preferred to range from 30 minutes to 120 minutes and more preferred to be 60 minutes; the said initial temperature is an arbitrary temperature value between the room temperature and the final temperature and is preferred to be 60°C; the said final temperature is a temperature value allowed by the adhesion conditions that can achieve the best adhesion performance of the adhesive under such adhesion conditions, including a certain optimum adhesion temperature between 60°C and 200°C obtained through practical experiments.

The use of catalyst A can accelerate the ring opening and crosslinking of the benzoxazine groups, reduce the reaction temperature and shorten the reaction time. Both the generalized lewis acids and the generalized lewis bases can promote the ring-opening process. Upon overall consideration, catalyst A is preferred to be benzenesulfonic acid, acetic acid and sodium hydroxide. A too large dosage of the catalyst A will lead to too fast crosslinking reaction, resulting in internal defects of the adhesive system. Therefore, the dosage of the catalyst shall not be excessive. Through comprehensive consideration, the dosage of the catalyst is preferred to be 0- 3 parts.

The benzoxazine adhesive for polyethylene prepared in the invention can be used directly as an adhesive. However, as performance of such adhesive is greatly affected by other organic groups, it can only achieve adhesion under specific conditions. The use of various additives (including various fillers and auxiliaries, etc.) to mix with the adhesive can greatly improve the comprehensive performance of the adhesive, so as to meet the adhesion demands of the various occasions. Examples are as follows:
The use of various fillers, especially reinforcement fillers, can improve the mechanical properties of the adhesive greatly. As the base polymer used in the invention contains both benzoxazine groups and phenolic hydroxyl groups, it can be mixed with the various inorganic fillers and organic polar fillers with easy blending, good compatibility, high reinforcement efficiency and other characteristics. In order to further improve the performance of the adhesive at high temperatures, the invention has further preferred the white carbon black treated with the phenyl group containing compounds for reinforcement, so as to slow down the softening and thermal degradation phenomena of the adhesive when used at high temperatures.

The addition of various auxiliaries, such as thermal oxygen stabilizer, flame retardant, conductive agent, vesicant, deep curing agent, pigment and plasticizer, to the formula involved in the invention can further improve the performance of the adhesive and expand its applications. Certain auxiliaries can also play a number of roles. For example, the iron red can serve as hot oxygen stabilizer, pigment and reinforcement filler at the same time. The addition of special polymers can improve the wettability of such kind of adhesives and specific interfaces. The addition of special coupling agents can improve the adhesive performance of this kind of adhesives and special interfaces. In short, by changing the dosage of additives and using a variety of additives together, a high performance adhesive can be produced to meet the needs of various occasions, with broad application prospects and good market prospects.

### Brief Description of the Figures

Figure 1 is the infrared spectrum of the benzoxazine adhesive with phenolic hydroxyl containing benzoxazine groups at the two ends obtained in Embodiment 6.
Figure 2 is the h-nuclear magnetic resonance spectrum of the benzoxazine adhesive with phenolic hydroxyl containing benzoxazine groups at the two ends obtained in Embodiment 6.

### Detailed Embodiments

The invention is further described as follows in combination with the specific embodiments, but is not limited to that.

The raw materials used in the embodiments are all conventional raw materials available on the market or can be prepared according to the methods in the references.

The molar ratios as described in the embodiments are proportions of the amount of substances. All proportions are expressed as parts by mass.

### Embodiment 1

N-butylamine, phloroglucinol and trioxymethylene are blended evenly in methylbenzene according to the molar ratio of 1:10:4; the temperature is maintained at 80°C and the mixture is mechanically agitated for 6 hours; the phenolic hydroxyl group-containing benzoxazine adhesive is then obtained upon purification with a reaction yield of 85%.

### Embodiment 2

As described in Embodiment 1, provided that the n-butylamine is changed to ethylenediamine, the trioxymethylene is changed to formaldehyde solution (37wt%), the phloroglucinol is changed to catechol, and their molar ratio is changed to 1:2:4; the reaction temperature is changed to 70°C; the mechanical agitation time is changed to 5 hours; and the benzoxazine adhesive with phenolic hydroxyl group-containing benzoxazine groups at the two ends is obtained, with a reaction yield of 95%.

### Embodiment 3

As described in Embodiment 2, provided that the ethylenediamine is changed to aminopropyl-terminated disiloxane, while the other parameters are maintained the same; and the benzoxazine adhesive of a siloxysilane structure with phenolic hydroxyl group-containing benzoxazine groups at the two ends is obtained, with a reaction yield of 89%.

### Embodiment 4

As described in Embodiment 3, provided that the aminopropyl-terminated disiloxane is changed to aminopropyl-terminated polysiloxane (number-average molar mass 10,000); and the benzoxazine adhesive of a polysiloxane containing structure with phenolic hydroxyl group-containing benzoxazine groups at the two ends is obtained, with a reaction yield of 94%.

### Embodiment 5

As described in Embodiment 3, provided that the aminopropyl-terminated disiloxane is changed to polysiloxane with aminopropyl groups in the side chain (aminopropyl unit content in the polysiloxane: 5%; number-average molar mass: 120,000); and finally, the polysiloxane with benzoxazine groups in the side chain is obtained, with a reaction yield of 90%.

### Embodiment 6

As described in Embodiment 3, provided that the aminopropyl-terminated disiloxane is changed to hexanediamine; the aldehyde source is paraformaldehyde; the phenol source is catechol; and the products thus prepared are straight chain aliphatic compounds with benzoxazine group-containing end groups with a reaction yield of 95%.

The structure of the benzoxazine adhesive obtained in this embodiment is as follows:

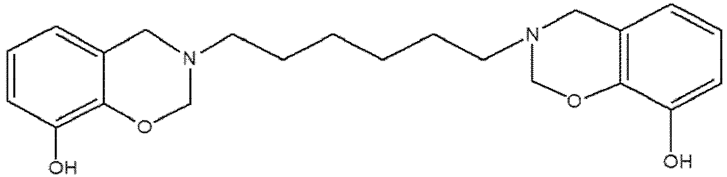

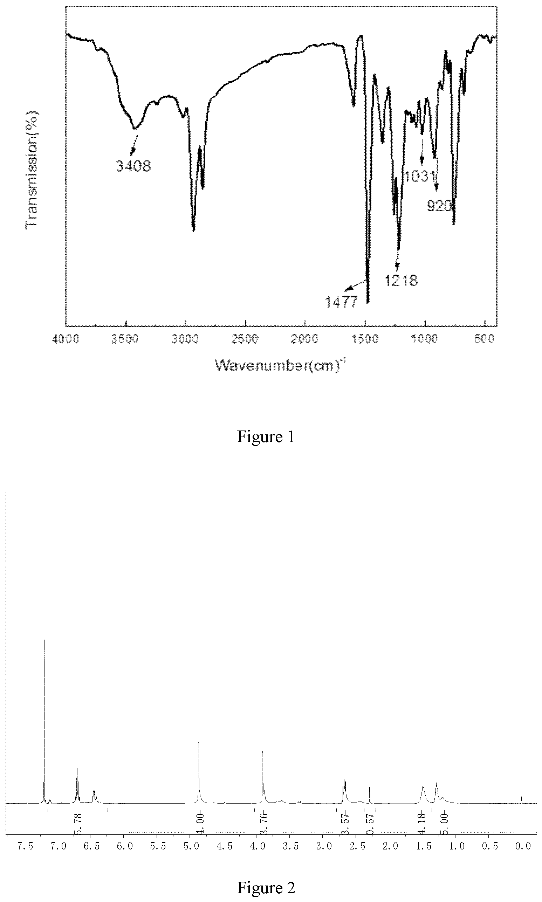

The infrared spectrum of the benzoxazine adhesive with phenolic hydroxyl containing benzoxazine groups at the two ends obtained in this embodiment is as shown in Figure 1. As can be seen from the Figure 1, the peaks at 1218cm⁻¹ and 1031cm⁻¹ are respectively the asymmetric stretching vibration peaks of the C-O-C and C-N-C on the oxazine ring. The peak at 920cm⁻¹ is the characteristic absorption peak of the oxazine ring connected with the benzene ring, namely a peak of the said oxazine ring, the absorption peak at 1477cm⁻¹ is a stretching vibration peak on the triple-substitution benzene ring, and the bulge peak at 3408cm⁻¹ is resulted in by the hydrogen bonds formed by the hydroxyl groups on the benzene ring.

The h-nuclear magnetic resonance spectrum of the benzoxazine adhesive obtained in this embodiment is as shown in Figure 2. The h-nuclear magnetic resonance spectrum data are as follows:
¹ HNMR(CDCl₃):δ(ppm)1.26(t,4H,CH₂NCH₂),1.48(m,CH₂CH₂N),2.26-2.67(m,4H,CH₂CH₂C H₂),3.84 (s, 4H,ArCH₂N), 4.82 (s, 4H, O-CH2-N), 6.3-7. 1(m,6H,Ar-H).

### Embodiment 7

As described in Embodiment 6, provided that the phenol source is changed from catechol to resorcinol; the ammonia source is silicon-containing dendrimer with amino groups as the end groups; the aldehyde source is paraformaldehyde; and the silicon-containing dendrimer with benzoxazine group-containing end groups is obtained with a reaction yield of 93%.

### Embodiment 8

As described in Embodiment 7, provided that the phenol source is changed from catechol to hydroquinol while the other parameters are maintained the same; and the product, silicon-containing dendrimer with benzoxazine group-containing end groups is obtained with a reaction yield of 96%.

### Embodiment 9

As described in Embodiment 8, provided that the silicon-containing dendrimer with amino groups as the end groups is changed to aminopropyl c silane; the paraformaldehyde is changed to formaldehyde solution; the solvent is changed to methylbenzene; and the product, benzoxazine group-containing dimethyl ethoxy silane is obtained with a reaction yield of 93%.

### Embodiment 10

As described in Embodiment 9, provided that the aminopropyl dimethyl ethoxy silane is changed to aminopropyl triethoxy silane; the hydroquinol is changed to o-hydroxybenzaldehyde; the solvent is changed to chloroform; and the product, benzoxazine group-containing triethoxy silane is obtained with a reaction yield of 96%.

### Embodiment 11

As described in Embodiment 10, provided that the aminopropyl triethoxy silane is changed to aminopropyl diethoxy methyl silane while the other parameters are maintained the same; acetic acid is added as the catalyst for the first step reaction and the dosage is 5% of the amount of aminopropyl diethoxy methyl silane; the reaction time is changed to 6h; and the product, benzoxazine group-containing silane is obtained, with a reaction yield of 91%.

### Embodiment 12

the benzoxazine adhesive of a siloxysilane structure with phenolic hydroxyl group-containing benzoxazine groups at the two ends obtained in Embodiment 3 is applied to the polyethylene plate, and then is cured under certain conditions (curing conditions: heating at 80°C for 1h; heating at 90°C for 1h; heating at 100°C for 1h; heating at 110°C for 1h; and heating at 120°C for 1h). The plate is kept still for 1 day after curing, and then is to be tested with a tensile machine.

### Embodiment 13

The benzoxazine adhesive with phenolic hydroxyl group-containing benzoxazine groups at the two ends obtained in Embodiment 2 is used as a basic formula component (100 parts) and then is mixed evenly with fumed silica (5 parts) and iron red (1 part); the mixture is then applied evenly to the polyethylene plate, and is cured under certain conditions (curing conditions: heating at 80°C for 1h; heating at 90°C for 1h; heating at 100°C for 1h; heating at 110°C for 1h; and heating at 120°C for 1h). The plate is kept still for 1 day after curing, and then is to be tested with a tensile machine.

### Embodiment 14

The benzoxazine adhesive of silicon-containing dendrimer structure with benzoxazine group-containing end groups obtained in Embodiment 6 is applied to the polyethylene plate, and then is cured under certain conditions (curing conditions: heating at 120°C for 1h; heating at 130°C for 1h; heating at 140°C for 1h; heating at 150°C for 1h; and heating at 160°C for 1h). The plate is kept still for 1 day after curing, and then is to be tested with a tensile machine.

### Embodiment 15

The adhesive of silicon-containing dendrimer with benzoxazine group-containing end groups obtained in Embodiment 7 is applied to the polyethylene plate, and then is cured under certain conditions (curing conditions: heating at 70°C for 1h; heating at 80°C for 1h; and heating at 90°C for 2h). The plate is kept still for 1 day before curing and another 1 day after curing, and then is to be tested with a tensile machine.

### Embodiment 16

The benzoxazine adhesive of benzoxazine group-containing triethoxy silane structure obtained in Embodiment 10 is used as a basic formula component and then is mixed evenly with acetic acid, M-5 white carbon black treated with silazane (15 parts) and silane coupling agent KH570 (3 parts); the mixture is then applied evenly to the steel plate, and is cured under certain conditions (curing conditions: heating at 90°C for 1h; heating at 100°C for 1h; heating at 110°C for 1h; and heating at 120°C for 1h). The plate is kept still for 1 day after curing, and then is to be tested with a tensile machine.

### Experimental Case

Adhesive strength data of the embodiments tested are as shown in Table 1.

**Table 1 Test results**

| S/N | Adhesive strength, MPa | | | |
|---|---|---|---|---|
| | 1 | 2 | 3 | Average |
| Embodiment 12 | 1.10 | 1.12 | 1.16 | 1.13 |
| Embodiment 13 | 1.21 | 1.27 | 1.24 | 1.24 |
| Embodiment 14 | 1.43 | 1.39 | 1.42 | 1.41 |
| Embodiment 15 | 1.31 | 1.29 | 1.36 | 1.32 |
| Comparative case 1* | - | - | - | 1.40 |
| Comparative case 2* | - | - | - | 1.20 |

| | | | | |
|---|---|---|---|---|
| *Note: Data of comparative case 1 come from the patent (publication number: CN 104531006 A). **Note: Data of comparative case 2 come from the patent (publication number: CN 104293256 A). | | | | |

As can be seen from Table 1, the phenolic hydroxyl group-containing benzoxazine adhesive prepared in the invention can be used as an adhesive with high strength directly. When the phenolic hydroxyl group-containing benzoxazine adhesive is mixed with other fillers and auxiliaries, its performance has been further improved and its adhesion strength is even higher than the tensile strength data of the polyethylene material coatings reported in the literatures. Such data show that the adhesive in the invention has obvious superiority in performance. Combined with the said advantages of the invention in the preparation process, the creativity of the invention is further demonstrated.

## Claims

1. An adhesive for polyethylene, **characterized in that** the adhesive is a phenolic hydroxyl group and long-chain alkyl group-containing benzoxazine compound.

2. The said adhesive for polyethylene according to Claim 1, **characterized in that** the said adhesive is a compound with the structure as shown in chemical formula (I): 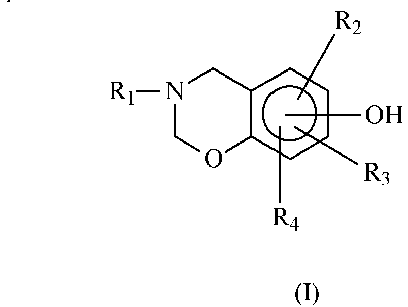 In chemical formula (I), R₁ is an organic group containing long-chain alkyl groups; R₂ to R₄ are various aliphatic hydrocarbon groups, silicon alkyl groups and/or aromatic hydrocarbon groups; or R₂ to R₄ are directly hydrogen atoms respectively.

3. The preparation method of the said adhesive for polyethylene according to Claim 1 comprises the steps as follows:
Under the conditions of with or without solvents, the amino group and long chain alkyl group-containing organic compound is mixed with the aldehyde (ketone) compound and the organic compound with no less than two phenolic hydroxyl groups in a benzene ring to react with each other to get a phenolic hydroxyl group- containing benzoxazine adhesive.

4. The said preparation method of the adhesive for polyethylene according to Claim 3, **characterized in that** the said amino group and long chain alkyl group-containing organic compound is an organic compound containing no less than one amino group and long-chain alkyl group simultaneously.

5. The said preparation method of the adhesive for polyethylene according to Claim 3, **characterized in that** the said amino group and long chain alkyl group-containing organic compound is amino-containing small molecule compound, amino-containing linear polymer, amino-containing comb-like macromolecule, amino-containing dendrimer or amino-containing composite material; and is preferred to be small molecule compound containing multiple amino groups, linear polymer with amino groups in the side chain and amino-containing dendrimer.

6. The said preparation method of the adhesive for polyethylene according to Claim 3, **characterized in that** the said aldehyde (ketone) compounds are all kinds of aldehyde (ketone) organic compounds that can be dissolved in this system.

7. The said preparation method of the adhesive for polyethylene according to Claim 3, **characterized in that** the said aldehyde (ketone) compound is formaldehyde, acetone, benzaldehyde or cyclohexanone.

8. The said preparation method of the adhesive for polyethylene according to Claim 3, **characterized in that** the said organic compound with no less than two phenolic hydroxyl groups in a benzene ring indicates all kinds of diphenol compounds, trihydric phenol compounds and tetrahydric phenol compounds.

9. The said preparation method of the adhesive for polyethylene according to Claim 3, **characterized in that** the said organic compound with no less than two phenolic hydroxyl groups in a benzene ring is catechol, resorcinol, hydroquinone, phloroglucinol and four phenolic group benzene.

10. The said preparation method of the adhesive for polyethylene according to Claim 3, **characterized in that** the said solvents are all kinds of polar or non-polar solvents that can dissolve the reactants, but do not react with the reactants, and are preferred to be methylbenzene, tetrahydrofuran, chloroform, methyl alcohol, diphenyl ether, dimethyl sulfoxide or N, N-dimethyl formamide.

11. The said preparation method of the adhesive for polyethylene according to Claim 3, **characterized in that** the molar ratio of the said amino-containing organic compound, aldehyde (ketone) compound and organic compound with no less than two phenolic hydroxyl groups in a benzene ring is 1: (0.1-15): (0.1-20);
The reaction temperature of the said amino-containing organic compound, aldehyde (ketone) compound and organic compound with no less than two phenolic hydroxyl groups in a benzene ring is 30-180°C.

12. A benzoxazine adhesive composition for polyethylene, **characterized in that** it comprises the following components in parts by mass:
100 parts of the said phenolic hydroxyl group and long-chain alkyl group-containing benzoxazine adhesive according to Claim 1, 0- 10 parts of catalyst A, 0-400 parts of filler and 0- 200 parts of auxiliaries.

13. The said benzoxazine adhesive composition for polyethylene according to Claim 12, **characterized in that** the said catalyst A is a compound that can catalyze the ring opening and polymerization reaction of the benzoxazine; the said filler is a variety of additives that can improve the performance of the benzoxazinyl adhesive; and the said auxiliaries are the various auxiliaries that do not significantly reduce the performance of the adhesive after being added.

14. The said benzoxazine adhesive composition for polyethylene according to Claim 12, **characterized in that** the said catalyst A is benzenesulfonic acid, acetic acid and sodium hydroxide; the said filler is fumed silica, precipitated silica, carbon black, calcium carbonate, aluminium hydroxide and/or aluminium hydroxide; and the said auxiliaries are thermal oxygen stabilizer, flame retardant, conductive agent, vesicant, deep curing agent, pigment and/or plasticizer.

15. The application method of the said benzoxazine adhesive for polyethylene according to Claim 1 comprises the following steps:
The adhesive can be used as an adhesive directly;
Or used together with other catalysts, fillers and auxiliaries.

## Amended claims

### Amended claims in accordance with Rule 137(2) EPC.

1. An adhesive for polyethylene, wherein the adhesive is a compound with the structure as shown in chemical formula (I): 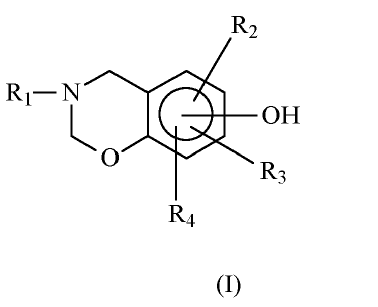 in chemical formula (I), R₁ is an organic group containing long-chain alkyl groups; R₂ to R₄ are various aliphatic hydrocarbon groups, silicon alkyl groups and/or aromatic hydrocarbon groups, or R₂ to R₄ are directly hydrogen atoms respectively,
**characterized in that** R₁ is a side chain of a linear polymer, or a group with dendrimer structure.

2. A preparation method of the adhesive for polyethylene according to claim 1 comprising the steps as follows:
under the conditions of with or without solvents, an amino group and long chain alkyl group-containing organic compound being mixed with an aldehyde (ketone) compound and an organic compound with no less than two phenolic hydroxyl groups in a benzene ring to react with each other to get a phenolic hydroxyl group-containing benzoxazine adhesive,
**characterized in that** the amino group and long chain alkyl group-containing organic compound is a linear polymer with amino groups in the side chain, or an amino-containing dendrimer.

3. The preparation method of the adhesive for polyethylene according to claim 2, wherein the aldehyde (ketone) compound is selected from all kinds of aldehyde (ketone) organic compounds that can be dissolved in this system.

4. The preparation method of the adhesive for polyethylene according to claim 2, wherein the aldehyde (ketone) compound is formaldehyde, acetone, benzaldehyde or cyclohexanone.

5. The preparation method of the adhesive for polyethylene according to claim 2, wherein the organic compound with no less than two phenolic hydroxyl groups in a benzene ring is selected from all kinds of diphenol compounds, trihydric phenol compounds and tetrahydric phenol compounds.

6. The preparation method of the adhesive for polyethylene according to claim 2, wherein the organic compound with no less than two phenolic hydroxyl groups in a benzene ring is catechol, resorcinol, hydroquinone, phloroglucinol or a four phenolic group benzene.

7. The preparation method of the adhesive for polyethylene according to claim 2, wherein the solvents are selected from all kinds of polar or non-polar solvents that can dissolve the reactants, but do not react with the reactants, and are preferred to be methylbenzene, tetrahydrofuran, chloroform, methyl alcohol, diphenyl ether, dimethyl sulfoxide or N, N-dimethyl formamide.

8. The preparation method of the adhesive for polyethylene according to claim 2, wherein the molar ratio of the amino-containing organic compound, the aldehyde (ketone) compound and the organic compound with no less than two phenolic hydroxyl groups in a benzene ring is 1: (0.1-15): (0.1-20);
the reaction temperature of the amino-containing organic compound, the aldehyde (ketone) compound and the organic compound with no less than two phenolic hydroxyl groups in a benzene ring is 30-180°C.

9. A benzoxazine adhesive composition for polyethylene, **characterized in that** it comprises the following components in parts by mass:
100 parts of the adhesive for polyethylene according to claim 1, 0-10 parts of catalyst A, 0-400 parts of filler and 0-200 parts of auxiliaries.

10. The benzoxazine adhesive composition for polyethylene according to claim 9, wherein the catalyst A is a compound that can catalyze ring opening and polymerization reaction of benzoxazine; the filler is selected from a variety of additives that can improve the performance of a benzoxazinyl adhesive; and the auxiliaries are various auxiliaries that do not significantly reduce the performance of the adhesive after being added.

11. The benzoxazine adhesive composition for polyethylene according to claim 9, wherein the catalyst A is benzenesulfonic acid, acetic acid or sodium hydroxide; the filler is fumed silica, precipitated silica, carbon black, calcium carbonate, aluminium hydroxide and/or aluminium hydroxide; and the auxiliaries are thermal oxygen stabilizer, flame retardant, conductive agent, vesicant, deep curing agent, pigment and/or plasticizer.

12. An application method of the adhesive for polyethylene according to claim 1 comprising the following steps:
using the adhesive as an adhesive directly;
or using the adhesive together with other catalysts, fillers and auxiliaries.
